# EUROPEAN PATENT SPECIFICATION

(11) **EP 4 559 498 B1**
(45) Date of publication and mention of the grant of the patent: **20.05.2026**
(21) Application number: 24213846.9
(22) Date of filing: 19.11.2024
(51) Int. Cl.: A61M 15/00

(54) **ACCESSORY FOR AEROSOL THERAPY AND METHOD OF MANUFACTURING THE SAME**
ZUBEHÖR FÜR DIE AEROSOL-THERAPIE UND VERFAHREN ZU SEINER HERSTELLUNG
ACCESSOIRE POUR LA THERAPIE AEROSOLIQUE ET MÉTHODE POUR SA FABRICATION

(30) Priority: 23.11.2023 IT 202300024945
(43) Date of publication of application: 28.05.2025
(73) Proprietor: 3A Health Care S.R.L., 25017 Lonato del Garda, BRESCIA (IT)
(72) Inventor: ABATE, Simone, 25017 Lonato del Garda, BRESCIA (IT); FRACCAROLI, Davide, 25017 Lonato del Garda, BRESCIA (IT)
(74) Representative: Chimini, Francesco

(56) References cited:
- WO-A1-01/05458
- WO-A1-2020/198736
- WO-A1-2023/010180
- KR-B1- 100 950 451
- KR-U- 20120 002 133
- US-A1- 2009 032 019
- US-A1- 2015 174 354
- US-A1- 2020 101 247

## Description

The present invention relates to the field of aerosol therapy treatments for the respiratory tract, which use a nebulizer ampoule that is suitable for generating an aerosol of a medical substance.

As is known, depending upon the treatment required, several inhalation accessories may be attached to the nebulizer ampoule. In particular, the nebulization ampoule is provided with an outlet mouthpiece whereto an aerosol therapy mask may be attached for inhalation from both the nose and the mouth, a mouthpiece for inhalation from the mouth only, and a nasal accessory, usually provided with a pair of tubes that are suitable for insertion into the nostrils of the user for inhalation from the nose only.

Such inhalation accessories, once in use, must be thoroughly washed and/or sterilized before subsequent use. The sterilization of the inhalation accessory is particularly important when the treatment of the respiratory tract is carried out within a medical facility, such as a hospital or clinic. US 2009/032019 A1 discloses a disposable aerosol therapy mask made from a flat sheet, including locking means such as tabs and slots. Further aerosol therapy accessories and related systems are known from US 2015/174354 A1, WO 01/05458 A1, WO 2023/010180 A1, and KR 100 950 451 B1.

One object of the present invention is to propose an alternative embodiment of an inhalation accessory, with respect to commonly used accessories, which in particular solves the problem of washing or sterilizing the accessory after use.

A further object of the invention is to propose a mask or mouthpiece that is simple and inexpensive to manufacture, and that is also of reduced dimensions when not in use.

This object is achieved with a method for making a mask or mouthpiece according to claim 1 and with a mask or mouthpiece according to claims 8 and 9.

The dependent claims describe preferred or advantageous embodiments of the invention.

The features and advantages of the invention will become apparent from the description below of the preferred embodiments thereof, given by way of nonlimiting example, with reference to the accompanying drawings, wherein:
- Fig. 1 is a plan view of the shape representing the flat development of an aerosol therapy mask;

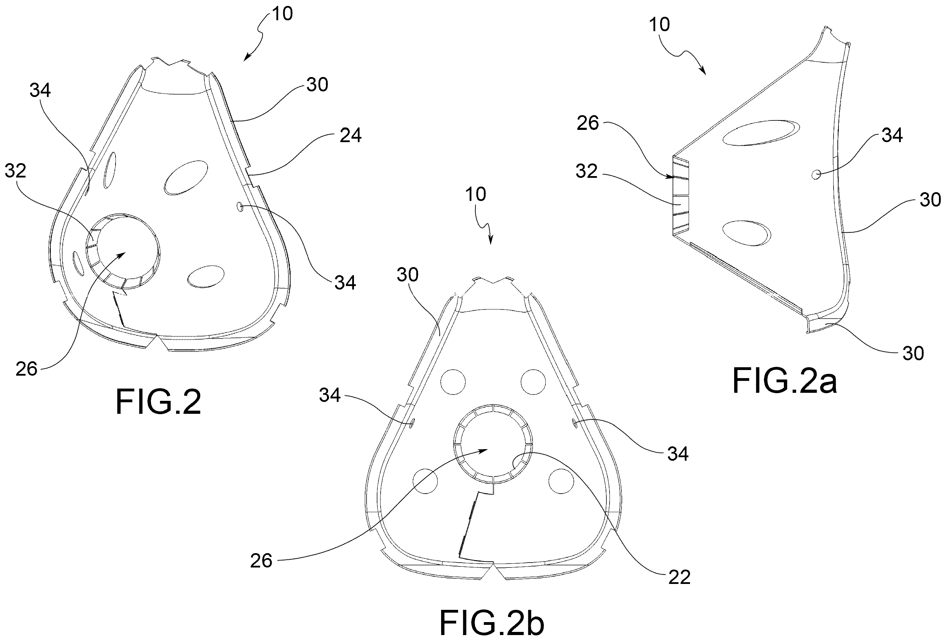

- Fig. 2, 2a and 2b are a perspective view, a side view, and a front view, respectively, of the shape of Fig. 1 assembled in the usage configuration;
- Fig. 3 and 3a are a perspective view and a side view, respectively, of the assembled mask and of a tubular connection element for connecting to a nebulization ampoule, before the coupling thereof;
- Fig. 4 is a sectional view of the mask connected to the tubular element;
- Fig. 5 is a perspective view of the mask when being coupled to the tubular connection element connected to the outlet of a nebulization ampoule;
- Fig. 6 is a plan view of the shape representing the flat development of a mouthpiece that may be connected to a nebulization ampoule;
- Fig. 7 and 7a are a perspective view and a side view, respectively, of the mouthpiece shape of Fig. 6 assembled in the usage configuration;
- Fig. 8 and 8a are a perspective view and a side view, respectively, of the assembled mouthpiece and a tubular connection element for connecting to a nebulization ampoule, before the coupling thereof;
- Fig. 9 is a sectional view of the mouthpiece connected to the tubular connection element; and
- Fig. 10 is a perspective view of the mouthpiece connected to a nebulization ampoule by means of the tubular connection element.

In the remainder of this explanation, all directional references (for example, upper, lower, upward, downward, left, right, towards the left, towards the right, at the top, at the bottom, above, below, vertical, horizontal, clockwise and counterclockwise) are used only for identification purposes in order to help the reader understand the described embodiments and do not create limitations, in particular with regard to the position, orientation or use of the described embodiments.

The joining references (e.g. fixed, coupled, connected, and the like) should be interpreted broadly and may include intermediate elements between a connection of elements and a relative movement between elements. The joining references do not therefore necessarily imply that two elements are directly connected in a fixed relationship with one another.

With reference to the accompanying drawings, the numerals 10 and 100 indicate, respectively, a mask and a mouthpiece which may be connected to a nebulization ampoule 200 for the treatment of the respiratory tract by aerosol therapy.

The mask 10 and the mouthpiece 100 are obtained from a respective mask shape 12 and mouthpiece shape 112 corresponding to the flat development of the mask 10 and the mouthpiece 100.

The shapes 12; 112 are obtained from a sheet of cellulose-based material. For example, the cellulose-based material is paper or cardboard, preferably paper obtained from wood fiber originating from PEFC-certified forests.

The paper or cardboard has a weight such that the inhalation accessory is sufficiently rigid when, for the use thereof, it is applied to a nebulization ampoule and placed in contact with the face of the user (in the case of a face mask) or held between the lips of the user (in the case of a mouthpiece). This stiffness characteristic must be maintained for the entire treatment period, also in consideration of the fact that the paper or cardboard remains in contact with the aerosol for a few minutes.

For example, the shapes 12; 112 may be obtained by cutting or die-cutting the sheet.

The shapes of the mask 12 and mouthpiece 112 also form locking means 14; 114 that are suitable for locking the respective shapes 12; 112 in an assembled usage configuration.

Such assembled usage configuration is obtained by bringing together two opposite sides 16a, 16b; 116a, 116b of the shapes 12; 112. In other words, the shapes of the mask 12 and mouthpiece 112 are suitable for being wound about a rotation axis X; Y in such a way as to close over themselves. For example, in the final assembled configuration, end portions terminating with the respective two opposite sides 16, 16b; 116a, 116b are superimposed upon each other.

In one embodiment, the locking means 14; 114 comprise at least one tab 18; 118 extending from a first side 16a; 116a of the shape and at least one cut 20; 120 obtained in proximity to a second side 16b; 116b of the shape, opposite the first. The at least one tab 18; 118 is suitable for being inserted into the at least one cut 20; 120. The coupling between the tab and the respective cut, possibly rendered even more stable by bending the tab after it has passed through the cut, ensures sufficient interference for preventing the shape from returning to the flat starting position.

In one embodiment shown in the drawings, at least two cuts 20; 120, suitable for being engaged by respective tabs, are obtained in proximity to the second side 16b; 116b of the shape.

Furthermore, at least two cuts 20; 120 that are substantially parallel to each other and to the second side are obtained in proximity to the second side 16b; 116b of the shape. The at least one tab 18; 118 is selectively inserted into one of said two cuts in such a way as to form the mask 10 or mouthpiece 100 in one of two usage configurations of different widths, respectively, of the opening of the mask turned towards the face of the user or of the diameter of the mouthpiece.

In other words, by making several parallel rows of cuts 20 in the mask shape 12 it is possible, with a single shape, to obtain masks of different sizes, for example for a child and for an adult.

As regards the mouthpiece 100, by making several parallel rows of cuts 120 in the mouthpiece shape 112 it is possible, with a single shape, to obtain mouthpieces 100 of different diameters in order to adapt to different outlet mouths of the nebulizer ampoule.

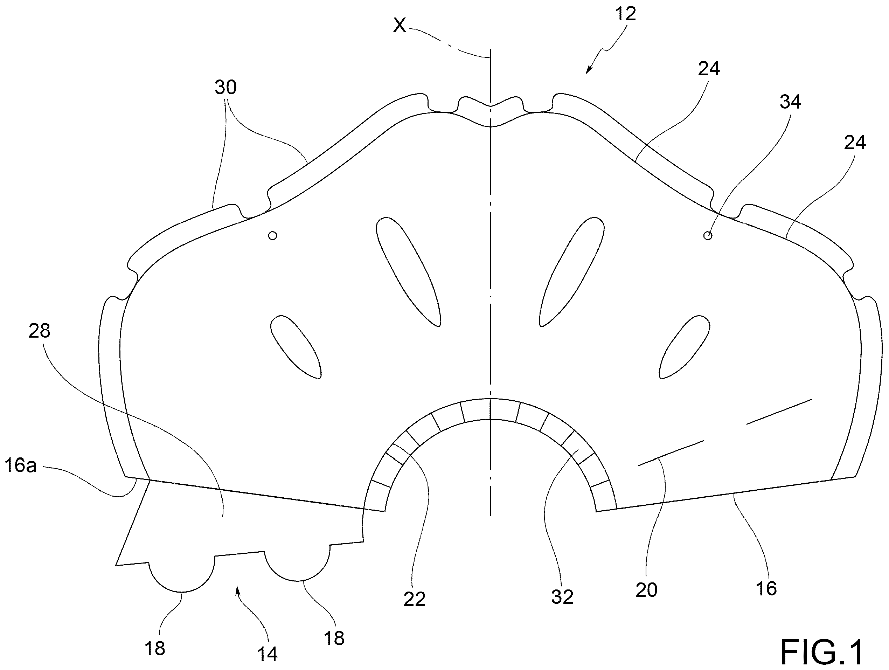

With reference now to Fig. 1, the mask shape 12 is defined by an inner edge 22, a first and a second side 16, 16b, and an outer edge 24.

The inner edge 22 has a substantially semicircular shape and is suitable for forming, in a usage configuration, a circular opening 26 for connection to the nebulizer ampoule 200.

The first side 16a and the second side 16b each extend from a respective end of the inner edge 22 in substantially opposite radial directions in order to form the two opposite sides of the mask shape 12.

The outer edge 24 extends between the distal ends of the first and second sides 16, 16b and is configured to form, in a usage configuration, the outer edge 24 of the mask that is suitable for resting on the face of the user.

At least one locking tab 18 extends from the first side 16a.

In other words, the first side 16a does not have an entirely straight course but, at least within an intermediate portion thereof, is shaped in such a way as to form at least one locking tab 18.

In the example in Fig. 1, the first side 16a forms, within an intermediate portion thereof, a locking wing 28 extending perpendicularly outwards with respect to the development direction of the first side 16a. Such locking wing 28, in turn, has an outer edge that is shaped in such a way as to form at least one, preferably two, locking tabs 18.

In one embodiment, the outer edge 24 of the shape of the mask 12, which, in the usage configuration, forms the edge of the mask which is suitable for resting on the face of the user, is configured in such a way as to form a plurality of foldable perimeter flaps 30 that are suitable for flexing upon contact with the face of the user.

Such foldable perimeter flaps 30, i.e., yielding, improve comfort when using the mask. In particular, preventing the edge of the mask, being thin insofar as it is made from a sheet, from marking the face of the user.

In one embodiment, in the inner edge 22 of the mask shape 12, i.e., the edge suitable for forming, in the usage configuration, the circular opening 26 for connection to the nebulizer ampoule 200, a crown 32 is obtained of pre-cut radial lines that is suitable for increasing the width of said circular connection opening. Depending, in fact, upon the diameter of the opening of the nebulization ampoule whereupon the mask is to be fitted, it is possible to maintain the crown 32 of pre-cut lines intact or, if such opening of the ampoule has a larger diameter, to break the pre-cut lines in such a way as to widen the connection opening 26.

In one embodiment, holes 34 are also obtained in the shape of the mask 12 for connecting to an elastic suitable for securing the mask 10 to the head of the patient.

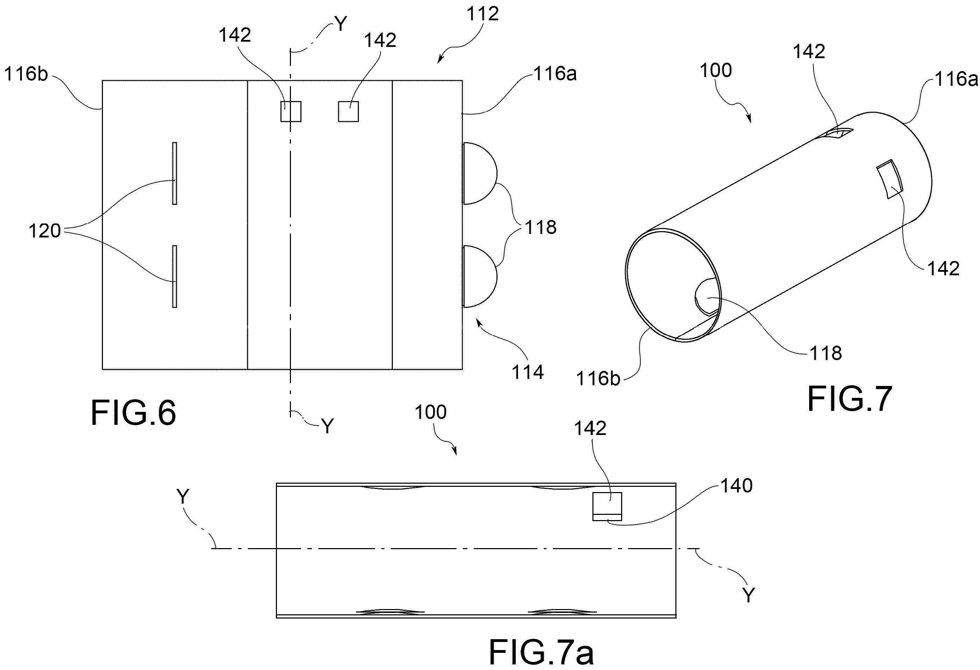

With reference now to Fig. 6, in one embodiment, at least one nasal inhalation opening 140, preferably two adjacent to each other, is formed in the shape of the mouthpiece 112, that is suitable for directing the flow of aerosol to the nostrils of the user when the open distal end of the mouthpiece 100 is plugged, for example by the closed lips of the user. Each nasal inhalation opening 140 may be engaged by a closing flap 142 extending from one side of the respective nasal opening 140. In this way, when the closing flap 142 is left in the engagement position of the relevant nasal opening 140, the flow of the nebulized substance reaches almost completely the open distal end of the mouthpiece and may be inhaled through the mouthpiece of the user. On the other hand, when the closing flap 142 is folded in such a way as to free the respective nasal opening 140 and the distal opening is closed, the nasal openings 140, if facing upwards, i.e. towards the nostrils of the user, allow the mouthpiece to be transformed into a nasal accessory, i.e., for inhaling the nebulized substance through the nostrils of the user.

In one embodiment, the shape of the mouthpiece 112 has a rectangular shape in such a way as to obtain a substantially cylindrical mouthpiece in the assembled usage configuration. On one side 116a of such rectangular shape, there extends at least one locking tab 118, preferably a pair of locking tabs 118.

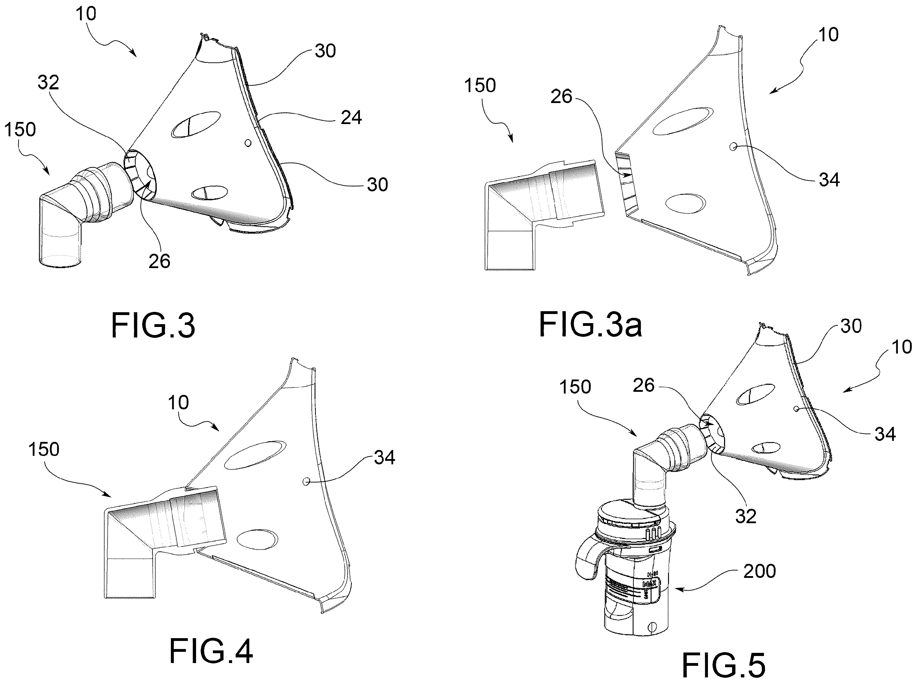

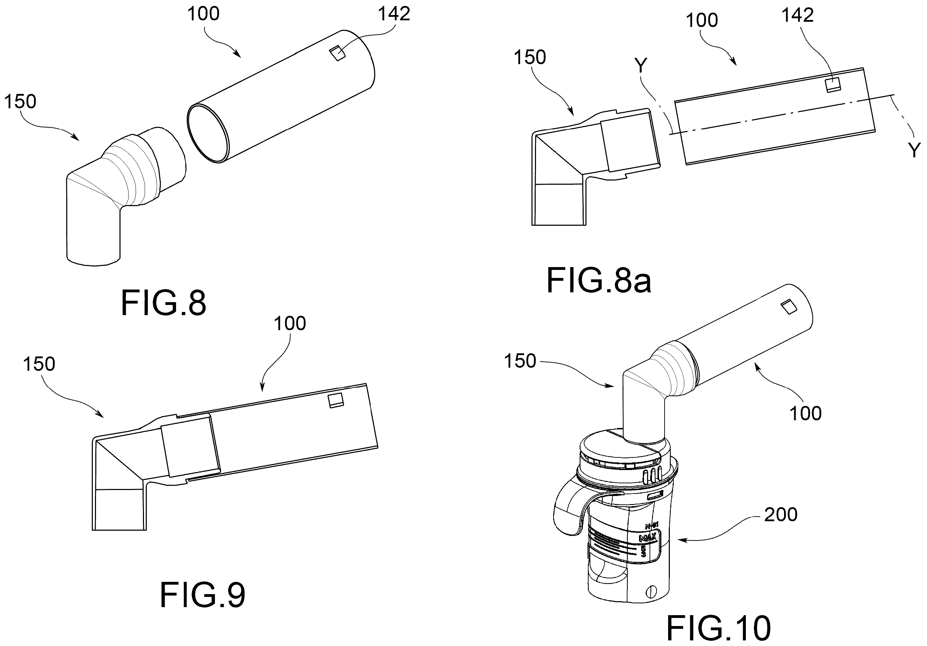

Fig. 3-5 and 8-10 show the mask 10 and the mouthpiece 100, in a usage configuration, coupled to an open distal end of a tubular connection element 150 having a proximal end which may be connected to an outlet of the nebulization ampoule 200.

The present invention also relates therefore to a kit for performing aerosol therapy treatments, comprising a nebulization ampoule 200, a tubular connection element 150 and a mask 10 and/or a mouthpiece 100 as described above.

It should be noted that the mask shape 12 and/or the mouthpiece shape 112 may be provided to the user in a still flat shape, in such a way as to optimize transport and storage, or else already preassembled.

Furthermore, the mask shape 12 and the mouthpiece shape 112 may be provided to the user in a flat shape and connected to each other, in such a way as to further optimize transport and storage.

In any case, the mask 10 and the mouthpiece 100 are disposable accessories made of a completely recyclable material. It is therefore not necessary to perform washing and sterilization operations after each use, furthermore completely eliminating any risk of the transmission of viruses and bacteria.

A person skilled in the art may make several changes, adjustments, adaptations, and replacements of elements with others that are functionally equivalent to the embodiments of the mask and mouthpiece for aerosol therapy according to the invention in order to meet incidental needs without departing from the scope of the following claims. Each of the features described as belonging to a possible embodiment may be obtained independently of the other described embodiments.

## Claims

1. Method for making a mask (10) or a mouthpiece (100) that may be connected to a nebulization ampoule (200) for carrying out aerosol therapy treatments, comprising the steps of:
- providing a cellulose-based sheet;
- cutting out, from said sheet, a shape (12; 112) corresponding to the flat development of the mask or mouthpiece, said shape (12; 112) comprising locking means (14; 114);
- forming the mask (10) or mouthpiece (100) by bringing two opposite sides (16a, 16b; 116a; 116b) of the shape together, said locking means being suitable for locking the mask or mouthpiece in the formed usage configuration, wherein said locking means (14; 114) comprise at least one tab (18; 118) extending from a first side of the shape and at least one cut (20; 120) obtained in proximity to a second side of the shape, opposite to the first, the at least one tab being suitable for insertion into the at least one cut,
**characterized in that** the at least one cut is at least two cuts (20; 120), substantially parallel to each other, obtained in proximity to the second side (16b; 116b) of the shape, the at least one tab (18; 118) being selectively insertable into one of said at least two cuts (20; 120) so as to form the mask or mouthpiece in one of at least two usage configurations of different widths, respectively, of the opening of the mask turned towards the face of the user or of the diameter of the mouthpiece.

2. Method according to claim 1, wherein the shape of the mask (112) is defined by:
- an internal edge (22) of substantially semicircular shape, said first internal edge (22) being suitable to form, in the usage configuration, a circular opening (26) for connection to the nebulization ampoule (200);
- a first side (16a) and a second side (16b), each of which extends from a respective end of the internal edge (22) in substantially opposite radial directions so as to form the two opposite sides of the shape;
- an external edge (24) which extends between the distal ends of the first and second sides and which is configured to form, in the usage configuration, the edge of the mask suitable for resting on the face of the user.

3. Method according to claim 2, wherein the external edge (24) of the shape of the mask which forms, in the usage configuration, the edge of the mask suitable for resting on the face of the user, is configured in such a way as to form a plurality of foldable perimeter flaps (30) suitable for flexing upon contact with the face of the user.

4. Method according to any of the preceding claims, wherein, in an internal edge (22) of the shape of the mask that is suitable for forming, in the usage configuration, a circular opening (26) for connection to the nebulization ampoule, a crown (32) is obtained of pre-cut radial lines, said crown being suitable for allowing the width of said circular opening (26) to be increased.

5. Method according to any of the preceding claims, wherein holes (34) are obtained in the shape of the mask (12) for connection to an elastic suitable for securing the mask to the head of the patient.

6. Method according to any of the preceding claims, wherein at least one nasal inhalation opening (140) suitable for directing the flow of aerosol towards the nostrils of the user is obtained inside the shape of the mouthpiece (112) when the open distal end of the mouthpiece is plugged, said nasal inhalation opening being engageable by a closing flap (142) which extends from one side of said nasal opening so as to transform the mouthpiece into a nasal accessory by disengaging the flap closing from the nasal opening.

7. Method according to any of the preceding claims, wherein the shape of the mouthpiece is rectangular, wherein at least one locking tab (118) extends from one side (116a) of the rectangular shape.

8. Mask connectable to a nebulization ampoule for carrying out aerosol therapy treatments, obtained by means of a manufacturing method according to any of claims 1-7.

9. Mouthpiece connectable to a nebulization ampoule for carrying out aerosol therapy treatments, obtained by a manufacturing method according to any of claims 1 or 6.

10. Flat shape (12; 112) obtained from a sheet of cellulose-based material and corresponding to the flat development of a mask (10) or mouthpiece (100) suitable for being connected to a nebulization ampoule (200) for carrying out aerosol therapy treatments, in said flat shape (12; 112) there being obtained locking means (14; 114), configured so as to lock the shape in a usage configuration wherein two opposite sides (16a, 16b; 116a, 166b) of the shape are brought together,
wherein said locking means (14; 114) comprise at least one tab (18; 118) extending from a first side of the shape and at least one cut (20; 120) obtained in proximity to a second side of the shape, opposite to the first, the at least one tab being suitable for insertion into the at least one cut,
**characterized in that** said at least one cut is at least two cuts (20; 120), substantially parallel to each other, obtained in proximity to the second side (16b; 116b) of the shape, the at least one tab (18; 118) being selectively insertable into one of said at least two cuts (20; 120) so as to form the mask or mouthpiece in one of at least two usage configurations of different widths respectively, of the opening of the mask turned towards the face of the user or of the diameter of the mouthpiece.

11. Kit for carrying out aerosol therapy treatments, comprising a nebulization ampoule (200), a tubular connection element (150) having a proximal end connectable to an outlet of the nebulization ampoule and an open distal end, and a mask (10) and/or a mouthpiece (100) in accordance with claims 8 and/or 9, that may be connected to said open distal end of the tubular connection element.

## Patentansprüche

1. Verfahren zur Herstellung einer Maske (10) oder eines Mundstücks (100), die mit einer Verstäubungsampulle (200) verbunden werden kann, um Aerosoltherapiebehandlungen durchzuführen, umfassend die folgenden Schritte:
- Bereitstellen einer Folie, die auf Zellulose basiert;
- Schneiden aus der Folie, einer Form (12; 112), die der flachen Entwicklung der Maske oder des Mundstücks entspricht, wobei die Form (12; 112) Verriegelungsmittel (14; 114) umfasst;
- Bilden der Maske (10) oder des Mundstücks (100) durch Zusammenbringen zwei gegenüberliegender Seiten (16a, 16b; 116a; 116b) der Form, wobei die Verriegelungsmittel geeignet sind, die Maske oder das Mundstück in der gebildeten Gebrauchskonfiguration zu verriegeln, wobei die Verriegelungsmittel (14; 114) mindestens eine Lasche (18; 118), die sich von einer ersten Seite der Form erstreckt, und mindestens einen Einschnitt (20; 120) umfassen, der in Nähe einer zweiten Seite der Form, die der ersten gegenüberliegt, erhalten wird, wobei die mindestens eine Lasche geeignet ist, in den mindestens einen Einschnitt eingesetzt zu werden,
**dadurch gekennzeichnet, dass** der mindestens einen Einschnitt mindestens zwei Einschnitte (20; 120) ist, die im Wesentlichen parallel zueinander sind, die in Nähe der zweiten Seite (16b; 116b) der Form erhalten werden, wobei die mindestens eine Lasche (18; 118) selektiv in einen der mindestens zwei Einschnitte (20; 120) eingesetzt werden kann, um die Maske oder das Mundstück in einer von mindestens zwei Gebrauchskonfigurationen unterschiedlicher Breite zu bilden, jeweils von der Öffnung der Maske, die zu dem Gesicht des Benutzers gerichtet wird, oder des Durchmessers des Mundstücks.

2. Verfahren nach Anspruch 1, wobei die Form der Maske (112) durch:
- einen inneren Rand (22) von im Wesentlichen halbkreisförmiger Gestalt, wobei der erste innere Rand (22) geeignet ist, in der Gebrauchskonfiguration eine kreisförmige Öffnung (26) zur Verbindung mit der Verstäubungsampulle (200) zu bilden;
- eine erste Seite (16a) und eine zweite Seite (16b), von denen jede sich von einem jeweiligen Ende des inneren Randes (22) in im Wesentlichen entgegengesetzten radialen Richtungen erstreckt, um die zwei gegenüberliegenden Seiten der Form zu bilden;
- einen äußeren Rand (24), der sich zwischen den distalen Enden der ersten und der zweiten Seite erstreckt und der dazu konfiguriert ist, in der Gebrauchskonfiguration den Rand der Maske zu bilden, der geeignet ist, auf dem Gesicht des Benutzers aufzuliegen, definiert wird.

3. Verfahren nach Anspruch 2, wobei der äußere Rand (24) der Form der Maske, der in der Gebrauchskonfiguration den Rand der Maske bildet, der geeignet ist, auf dem Gesicht des Benutzers aufzuliegen, dazu konfiguriert ist, eine Mehrzahl von faltbaren Umfangslaschen (30) zu bilden, die geeignet sind, sich bei Kontakt mit dem Gesicht des Benutzers zu biegen.

4. Verfahren nach einem der vorhergehenden Ansprüche, wobei in einem inneren Rand (22) der Form der Maske, der geeignet ist, in der Gebrauchskonfiguration eine kreisförmige Öffnung (26) zur Verbindung mit der Verstäubungsampulle zu bilden, eine Krone (32) aus vorgeschnittenen radialen Linien erhalten wird, wobei die Krone geeignet ist, zu erlauben, die Breite der kreisförmigen Öffnung (26) zu vergrößern.

5. Verfahren nach einem der vorhergehenden Ansprüche, wobei Bohrungen (34) in der Form der Maske (12) zur Verbindung mit einem Gummiband erhalten werden, das geeignet ist, die Maske an dem Kopf des Patienten zu befestigen.

6. Verfahren nach einem der vorhergehenden Ansprüche, wobei mindestens eine Naseninhalationsöffnung (140), die geeignet ist, den Aerosolstrom zu den Nasenlöchern des Benutzers zu leiten, innerhalb der Form des Mundstücks (112) erhalten wird, wenn das offene distale Ende des Mundstücks verschlossen ist, wobei die Naseninhalationsöffnung durch eine Verschlusslasche (142) in Eingriff gebracht werden kann, die sich von einer Seite der Nasenöffnung erstreckt, um das Mundstück in ein Nasenzubehör umzuwandeln, indem die Verschlusslasche von der Nasenöffnung gelöst wird.

7. Verfahren nach einem der vorhergehenden Ansprüche, wobei die Form des Mundstücks rechteckig ist, wobei mindestens eine Verriegelungslasche (118) sich von einer Seite (116a) der rechteckigen Form erstreckt.

8. Maske, die mit einer Verstäubungsampulle verbindbar ist, um Aerosoltherapiebehandlungen durchzuführen, die mittels einen Herstellungsverfahrens nach einem der Ansprüche 1 bis 7 erhalten wird.

9. Mundstück, das mit einer Verstäubungsampulle verbindbar ist, um Aerosoltherapiebehandlungen durchzuführen, das mittels einen Herstellungsverfahrens nach einem der Ansprüche 1 oder 6 erhalten wird.

10. Flachform (12; 112), die aus einer Folie aus Material auf Zellulosebasis erhalten wird und die der flachen Entwicklung einer Maske (10) oder eines Mundstücks (100) entspricht, die zur Verbindung mit einer Verstäubungsampulle (200) geeignet ist, um Aerosoltherapiebehandlungen durchzuführen, wobei in der Flachform (12; 112) Verriegelungsmittel (14; 114) erhalten werden, die dazu konfiguriert sind, die Form in einer Gebrauchskonfiguration zu verriegeln, wobei zwei gegenüberliegende Seiten (16a, 16b; 116a, 166b) der Form zusammengebracht werden,
wobei die Verriegelungsmittel (14; 114) mindestens eine Lasche (18; 118) umfassen, die sich von einer ersten Seite der Form erstreckt, und mindestens einen Einschnitt (20; 120), der in der Nähe einer zweiten Seite der Form, die der ersten gegenüberliegt, erhalten wird, wobei die mindestens eine Lasche geeignet ist, in den mindestens einen Einschnitt eingesetzt zu werden,
**dadurch gekennzeichnet, dass** der mindestens einen Einschnitt mindestens
zwei Einschnitte (20; 120) ist, die im Wesentlichen parallel zueinander sind, die in der Nähe der zweiten Seite (16b; 116b) der Form erhalten werden, wobei die mindestens eine Lasche (18; 118) selektiv in einen der mindestens zwei Einschnitte (20; 120) eingesetzt werden kann, um die Maske oder das Mundstück in einer von mindestens zwei Gebrauchskonfigurationen von unterschiedlicher Breite zu bilden, jeweils von der Öffnung der Maske, die zu dem Gesicht des Benutzers gerichtet ist, oder des Durchmessers des Mundstücks.

11. Kit zur Durchführung von Aerosoltherapiebehandlungen, umfassend eine Verstäubungsampulle (200), ein rohrförmiges Verbindungselement (150) aufweisend ein proximales Ende, das mit einem Ausgang der Verstäubungsampulle verbindbar ist, und einem offenen distalen Ende, sowie eine Maske (10) und/oder ein Mundstück (100) nach den Ansprüchen 8 und/oder 9, die mit dem offenen distalen Ende des rohrförmigen Verbindungselements verbunden werden können.

## Revendications

1. Procédé pour la fabrication d'un masque (10) ou d'un embout buccal (100) pouvant être connecté à une ampoule de nébulisation (200) pour réaliser des traitements de thérapie aérosolique, comprenant les étapes suivantes :
- fournir une feuille à base de cellulose ;
- découper, à partir de ladite feuille, une forme (12; 112) correspondant au développement à plat du masque ou de l'embout buccal, ladite forme (12; 112) comprenant des moyens de verrouillage (14; 114) ;
- former le masque (10) ou l'embout buccal (100) en rapprochant deux côtés opposés (16a, 16b; 116a; 116b) de la forme, lesdits moyens de verrouillage étant adaptés pour verrouiller le masque ou l'embout buccal dans la configuration d'utilisation formée, dans lequel lesdits moyens de verrouillage (14; 114) comprennent au moins une languette (18; 118) s'étendant d'un premier côté de la forme et au moins une découpe (20; 120) obtenue à proximité d'un second côté de la forme, opposé au premier, ladite au moins une languette étant adaptée pour être insérée dans ladite au moins une découpe,
**caractérisé en ce que** ladite au moins une découpe est au moins deux découpes (20; 120), sensiblement parallèles entre elles, obtenues à proximité du second côté (16b; 116b) de la forme, ladite au moins une languette (18; 118) étant sélectivement insérable dans l'une desdites au moins deux découpes (20; 120) de manière à former le masque ou l'embout buccal dans l'une d'au moins deux configurations d'utilisation de largeurs différentes, respectivement, de l'ouverture du masque tournée vers le visage de l'utilisateur ou du diamètre de l'embout buccal.

2. Procédé selon la revendication 1, dans lequel la forme du masque (112) est définie par :
- un bord interne (22) de forme sensiblement semi-circulaire, ledit premier bord interne (22) étant adapté à former, dans la configuration d'utilisation, une ouverture circulaire (26) pour la connexion à l'ampoule de nébulisation (200) ;
- un premier côté (16a) et un second côté (16b), chacun desquels s'étend à partir d'une extrémité respective du bord interne (22) dans des directions radiales sensiblement opposées de manière à former les deux côtés opposés de la forme ;
- un bord externe (24) qui s'étend entre les extrémités distales du premier et du second côté et qui est configuré pour former, dans la configuration d'utilisation, le bord du masque adapté à s'appuyer sur le visage de l'utilisateur.

3. Procédé selon la revendication 2, dans lequel le bord externe (24) de la forme du masque qui forme, dans la configuration d'utilisation, le bord du masque adapté à s'appuyer sur le visage de l'utilisateur, est configuré de manière à former une pluralité de rabats périmétriques pliables (30) adaptés à se fléchir au contact avec le visage de l'utilisateur.

4. Procédé selon l'une quelconque des revendications précédentes, dans lequel, dans un bord interne (22) de la forme du masque qui est adapté à former, dans la configuration d'utilisation, une ouverture circulaire (26) pour la connexion à l'ampoule de nébulisation, une couronne (32) de lignes radiales pré-découpées est obtenue, ladite couronne étant adaptée pour permettre d'augmenter la largeur de ladite ouverture circulaire (26).

5. Procédé selon l'une quelconque des revendications précédentes, dans lequel des trous (34) sont obtenus dans la forme du masque (12) pour la connexion à un élastique adapté pour fixer le masque à la tête du patient.

6. Procédé selon l'une quelconque des revendications précédentes, dans lequel au moins une ouverture d'inhalation nasale (140) adaptée pour diriger le flux d'aérosol vers les narines de l'utilisateur est obtenue à l'intérieur de la forme de l'embout buccal (112) lorsque l'extrémité distale ouverte de l'embout buccal est obturée, ladite ouverture d'inhalation nasale étant engageable par un rabat de fermeture (142) qui s'étend d'un côté de ladite ouverture nasale de manière à transformer l'embout buccal en accessoire nasal en désengageant le rabat de fermeture de l'ouverture nasale.

7. Procédé selon l'une quelconque des revendications précédentes, dans lequel la forme de l'embout buccal est rectangulaire, dans lequel au moins une languette de verrouillage (118) s'étend d'un côté (116a) de la forme rectangulaire.

8. Masque connectable à une ampoule de nébulisation pour réaliser des traitements de thérapie aérosolique, obtenu au moyen d'un procédé de fabrication selon l'une quelconque des revendications 1-7.

9. Embout buccal connectable à une ampoule de nébulisation pour réaliser des traitements de thérapie aérosolique, obtenu par un procédé de fabrication selon l'une quelconque des revendications 1 ou 6.

10. Forme plane (12; 112) obtenue à partir d'une feuille de matériau à base de cellulose et correspondant au développement à plat d'un masque (10) ou d'un embout buccal (100) adapté pour être connecté à une ampoule de nébulisation (200) pour réaliser des traitements de thérapie aérosolique, dans ladite forme plane (12; 112) étant obtenus des moyens de verrouillage (14; 114), configurés de manière à verrouiller la forme dans une configuration d'utilisation dans laquelle deux côtés opposés (16a, 16b; 116a, 166b) de la forme sont rapprochés,
dans lequel lesdits moyens de verrouillage (14; 114) comprennent au moins une languette (18; 118) s'étendant d'un premier côté de la forme et au moins une découpe (20; 120) obtenue à proximité d'un second côté de la forme, opposé au premier, ladite au moins une languette étant adaptée pour être insérée dans ladite au moins une découpe,
**caractérisé en ce que** ladite au moins une découpe est au moins deux découpes (20; 120), sensiblement parallèles entre elles, obtenues à proximité du second côté (16b; 116b) de la forme, ladite au moins une languette (18; 118) étant sélectivement insérable dans l'une desdites au moins deux découpes (20; 120) de manière à former le masque ou l'embout buccal dans l'une d'au moins deux configurations d'utilisation de largeurs différentes respectivement, de l'ouverture du masque tournée vers le visage de l'utilisateur ou du diamètre de l'embout buccal.

11. Kit pour réaliser des traitements de thérapie aérosolique, comprenant une ampoule de nébulisation (200), un élément de connexion tubulaire (150) ayant une extrémité proximale connectable à une sortie de l'ampoule de nébulisation et une extrémité distale ouverte, et un masque (10) et/ou un embout buccal (100) conformément aux revendications 8 et/ou 9, pouvant être connectés à ladite extrémité distale ouverte de l' élément de connexion tubulaire.
